# EUROPÄISCHE PATENTSCHRIFT

(11) **EP 2 127 618 B1**
(45) Veröffentlichungstag und Bekanntmachung des Hinweises auf die Patenterteilung: **02.11.2011**
(21) Anmeldenummer: 08157209.1
(22) Anmeldetag: 29.05.2008
(51) Int. Cl.: A61F 9/01

(54) **Lasersystem zum Ablatieren von Hornhaut**
Laser system to ablate cornea
Système laser destiné à l'ablation de la cornée

(43) Veröffentlichungstag der Anmeldung: 02.12.2009
(73) Patentinhaber: Schwind eye-tech-solutions GmbH & Co. KG, 63801 Kleinostheim (DE)
(72) Erfinder: Arba-Mosquera, Samuel, 63739, Aschaffenburg (DE)
(74) Vertreter: Laub, Alexander

(56) Entgegenhaltungen:
- EP-A- 1 702 595
- US-A- 6 099 522
- US-A1- 2005 021 011

## Beschreibung

Die Erfindung betrifft ein Lasersystem zum Ablatieren von Hornhaut an einem Patientenauge.

Ein solches Lasersystem umfasst eine Laserquelle. Die Laserquelle sendet im Betrieb Laserstrahlenpulse aus. Die Laserstrahlenpulse werden nicht unmittelbar auf das Patientenauge gelenkt. Vielmehr werden sie nach ihrem Austreten aus der Laserquelle, aber bevor sie aus dem Lasersystem austreten, durch geeignete optische Mittel verändert. Insbesondere wird den Laserstrahlenpulsen ein vorbestimmtes Intensitätsprofil verliehen, das sie nach dem Austreten aus der Laserquelle noch nicht haben. Nachdem die Laserstrahlenpulse die optischen Mittel verlassen haben, gelangen sie auf eine Zieleinrichtung. In der Zieleinrichtung werden die Laserstrahlenpulse in unterschiedliche Richtungen abgelenkt, die unterschiedlichen Stellen auf der Hornhaut des Patientenauges entsprechen. Sie können direkt von der Zieleinrichtung auf die Patientenhornhaut gelangen, häufig ist jedoch noch ein Spiegel nachgeordnet. Vor der Zieleinrichtung, aber nachdem die Laserstrahlenpulse die optischen Mittel bereits durchlaufen haben, in denen ein Teil von ihnen ausgekoppelt worden sein kann, lässt sich die Energie der Laserstrahlenpulse durch eine geeignete Einrichtung messen. Eine solche Einrichtung ist bisher zwischen den optischen Mitteln und der Zieleinrichtung gelegentlich bereitgestellt, wobei die von dieser Einrichtung gewonnenen Messwerte in einem Speicher abgelegt werden, so dass nach der Laserablationsbehandlung eine Information über die hierbei in den Laserstrahlenpulsen bereitgestellten Energien zur Verfügung steht.

Es auch bekannt, zusätzlich oder alternativ unmittelbar hinter der Laserquelle die aus dieser austretenden Laserstrahlenpulse einer Messung auf ihre Energie hin zu unterziehen. Eine diesbezügliche Einrichtung zum Messen der Energie wird üblicherweise unmittelbar mit der Laserquelle gekoppelt. Die Einrichtung zum Messen gehört hierbei zu einem Regelsystem, das die Laserleistung regelt. Eine Regelung aufgrund einer Energiemessung nach den optischen Mitteln ist nicht sinnvoll, weil die optischen Mittel nicht stets gleich arbeiten und beim Durchlaufen der optischen Mittel durch den Laserstrahlenpuls deren Energie verringert werden kann.

Wenn die Einrichtung zum Messen der Energie der Laserstrahlenpulse unmittelbar hinter der Laserquelle angeordnet ist, der Laserstrahl also lediglich die zur Laserquelle, die üblicherweise als ein geschlossenes Gerät ausgebildet ist, intrinsisch zugehörigen optischen Mittel, z. B. das Auskoppelfenster, durchlaufen hat, während optische Mittel außerhalb der Laserquelle nicht durchlaufen wurden, ist die Einrichtung zum Messen der Energie nicht geeignet, eine Aussage über die Energie der tatsächlich zur Hornhaut gelangenden Laserstrahlenpulse zu machen.

Aufgrund von Schwankungen in der Betriebsweise der Komponenten des Lasersystems, nämlich der Laserquelle selbst und auch der optischen Mittel kann es passieren, dass während einer Laserablation mehr Energie als vorgesehen auf die Patientenhornhaut gelangt. Es würde dann zuviel Material von der Hornhaut abgetragen werden. Dies ist nicht wünschenswert. Im Zweifel ist eine nicht ausreichende Korrektur von einer Fehlsichtigkeit des Patienten einer Überkorrektur vorzuziehen.

Die US 2005/021011 A1 beschreibt ein Laserablationsgerät mit einem Laser, dem ein räumlicher Modulator nachgeordnet ist. Dem räumlichen Modulator ist ein halbdurchlässiger Spiegel nachgeordnet, der unmittelbar vor dem zu behandelnden Patienten Strahlen zu einem Photodetektor hin auskoppelt. Der Photodetektor übermittelt seine Messsignale an einen Ausfallsicherheitscomputer, dessen Aufgabe es ist, das zuverlässige Ablaufen des Behandlungsalgorithmus zu überprüfen und gegebenenfalls einen Alarm auszugeben und einen Anhaltebefehl an den Laser zu geben.

Das US-Patent 6,099,522 beschreibt eine Laserarbeitsstation mit einem Laserstrahlenpulse abgebenden Laser Die Abgabe von Laserstrahlenpulsen wird beendet, wenn eine kritische Situation auftritt. Nach Umformung des Laserstrahls durch Strahlformungsoptiken gibt es eine Stelle, von der aus zu einem Pulsenergieübenrvacher hin Laserstrahlung ausgekoppelt ist. Dieser Pulsenergieüberwacher ist unmittelbar mit der Computersteuereinheit, die den Laser ansteuert, gekoppelt.

Die EP 1 792 595 A1 beschreibt, dass aufgrund von Messungen des Auges und insbesondere auch des Laserstrahls während der Augenchirurgie nahezu jegliche Art von Rückkopplung auf den Laserbetrieb möglich ist. Die Eigenschaft wird durch einen Sensor gemessen. Dieser kann ein Energiesensor sein. Aufgrund erhöhter Energie kann die Zahl der Laserpulse verringert werden, im Extremfall erfolgt ein Abbruch der Behandlung.

Es ist Aufgabe der Erfindung, dazu beizutragen, dass nicht mehr Material als vorab geplant von der Hornhaut eines Patientenauges abgetragen wird. Die Aufgabe wird durch ein Lasersystem mit den Merkmalen gemäß Patentanspruch 1 gelöst. Das erfindungsgemäße Lasersystem zum Ablatieren von Hornhaut an einem Patientenauge weist somit eine Laserquelle auf, die im Betrieb Laserstrahlenpulse aussendet, die vor ihrem Austreten aus dem Lasersystem durch optische Mittel verändert werden, und es weist eine Einrichtung zum Messen der Energie jedes aus den optischen Mitteln austretenden Laserstrahlenpulses auf. Erfindungsgemäß ist die Einrichtung zum Messen mit einer Steuereinrichtung gekoppelt (bevorzugt verbunden), welche ihrerseits mit der Laserquelle zum Übertragen von Steuerbefehlen an diese gekoppelt (bevorzugt verbunden) ist.

Durch die Kopplung der Einrichtung zum Messen mit einer derartigen Steuereinrichtung können die Messwerte unmittelbar den Betrieb der Laserquelle beeinflussen. Somit ist es möglich, korrektiv einzugreifen, wenn die Einrichtung zum Messen Energien der Laserstrahlenpulse erfasst, die außerhalb der gewünschten Energien liegen.

Ein Kriterium, das durch die mit den der Einrichtung zum Messen gekoppelten Steuereinrichtung herangezogen wird, ist die gesamte ins Patientenauge eingetragene Energie. So ist vorgesehen, dass die Steuereinrichtung ein Beenden des Betriebs der Laserquelle bewirkt, wenn die Summe der Energie sämtlicher seit einem Beginn einer Laserablation abgegebenen Laserstrahlenpulse einen vorbestimmten Schwellwert überschreitet. So kann es sein, dass die einzelnen Laserstrahlenpulse eine etwas höhere Energie haben als vorgesehen, dass diese höhere Energie aber noch verträglich ist, so dass der diesbezügliche Schwellwert nicht überschritten wird. Es wird dann allerdings insgesamt zuviel Material von der Hornhaut abgetragen, so dass von dem Plan abgewichen werden muss, welcher festlegt, wie viele Laserstrahlenpulse auf einzelne Stellen der Hornhaut gelangen. Statt dessen müssen weniger Laserstrahlenpulse auf die Hornhaut gesendet werden. Im einfachsten Fall wird einfach der Betrieb der Laserquelle unterbrochen.

Eine Steuereinrichtung, die Steuerbefehle an die Laserquelle abgibt, ist üblicherweise in herkömmlichen Lasersystemen vorhanden. Gemäß einer Alternative der Erfindung ist die Einrichtung zum Messen mit genau einer solchen Steuereinrichtung gekoppelt. Eine solche Steuereinrichtung ist herkömmlicherweise auch dazu ausgelegt, eine (den optischen Mitteln nachgeordnete und bevorzugt auch der Einrichtung zum Messen nachgeordnete) Zielrichtung anzusteuern, die in Abhängigkeit von den Steuerbefehlen die Laserstrahlenpulse nacheinander auf unterschiedliche Stellen lenkt. Es handelt sich hier letztlich um unterschiedliche Stellen des Austrittsfensters des Lasersystems, wobei diese unterschiedlichen Stellen unterschiedlichen Stellen der Hornhaut des Patienten entsprechen sollen. Vor dem Austrittsfenster kann die Zieleinrichtung die Laserstrahlenpulse noch auf einen Spiegel lenken, dementsprechend auf unterschiedliche Stellen eines solchen Spiegels.

Eine Steuereinrichtung der genannten Art steuert typischerweise die Laserquelle und Zieleinrichtung synchron an, und zwar gemäß einer zuvor definierten Tabelle. Die Tabelle gibt wieder, welche Stellen der Hornhaut des Patienten mit wie vielen Laserstrahlenpulsen bestrahlt werden sollen. Die Steuereinrichtung bewirkt, dass die Tabelle Laserstrahlenpuls für Laserstrahlenpuls abgearbeitet wird, und zwar nach einer vorbestimmten Systematik, bei der auch Zufallsvariablen eingesetzt werden können. Nun soll ja verhindert werden, dass zuviel Energie auf das Patientenauge gelangt. Ist die Energie der Laserstrahlenpulse (einzelner Laserstrahlenpulse oder im Schnitt) höher als vorgesehen, würde ein konsequentes Abarbeiten der Tabelle durch die Steuereinrichtung genau bewirken, dass zuviel Energie auf das Patientenauge gelangt. Dementsprechend ist die Steuereinrichtung dazu ausgelegt, aufgrund eines oder mehrer vorbestimmter Ergebnisse einer Messung durch die Einrichtung zum Messen von einer Steuerung gemäß der Tabelle abzuweichen, indem nämlich der Betrieb der Laserquelle vollständig beendet wird.

Bei einer zweiten Alternative der Erfindung, ist diejenige Steuereinrichtung, die mit der Einrichtung zum Messen gekoppelt ist, von der ansonsten bereitgestellten Steuereinrichtung verschieden. Die mit der Einrichtung zum Messen gekoppelte Steuereinrichtung kann ausschließlich der Aufgabe dienen, den Betrieb der Laserquelle zu unterbrechen.

Bei beiden Alternativen, was die Steuereinrichtung betrifft, kann alternativ oder zusätzlich zu dem genannten Kriterium ein Beenden des Betriebs der Laserquelle bewirkt werden, wenn die Energie zumindest eines vorbestimmten Teils der zuletzt abgegebenen Laserstrahlenpulse einen Schwellwert überschreitet. Beispielsweise kann ein Abschalten der Laserquelle bewirkt werden, wenn mehr als zwei der letzten zehn Laserstrahlenpulse eine zu hohe Energie hatten. Üblicherweise wird die Energie der Laserstrahlenpulse in Abhängigkeit von Eigenschaften des Patientenauges eingestellt, damit eine genau vorgegebene Menge von Hornhautmaterial durch die einzelnen Laserstrahlenpulse abgetragen wird. Haben einzelne Laserstrahlenpulse eine zu hohe Energie, wird möglicherweise nicht einfach nur mehr Material abgetragen, sondern das Material wird unsauber abgetragen, und es entstehen Rückstände etc. Das Beenden des Betriebs der Laserquelle kann sogar bewirkt werden, wenn nur der eine zuletzt abgegebene Laserstrahlenpuls eine zu hohe Energie hat.

Naturgemäß ist es auch möglich, die Tabelle aufgrund der Ergebnisse der Energiemessung zu ändern und die Laserablation dann noch gemäß der Änderung fortzuführen.

Ein nicht beanspruchtes Verfahren zum Betreiben eines Lasersystems zum Ablatieren von Hornhaut umfasst, dass eine Laserquelle zur Abgabe von Laserstrahlenpulsen gemäß einer Ablationszielvorschrift veranlasst wird. Die Ablationszielvorschrift umfasst ein so genanntes Ablationsprofil, d. h. eine Kontur, die der zu ablatierenden Hornhaut durch die Ablation verliehen werden soll. Üblicherweise wird die Ablationszielvorschrift durch die oben bereits genannte Tabelle ausgedrückt, die wiedergibt, wie viele Laserstrahlenpulse ("Schüsse") auf welche Stellen gesandt werden sollen. Die Laserstrahlenpulse werden durch optische Mittel umgeformt. Dann werden sie typischerweise einer Zieleinrichtung zugeführt, die bewirkt, dass die Laserpulse zur Erreichung des Ablationsziels nacheinander auf unterschiedliche Stellen gelenkt werden, wobei es sich um unterschiedliche Stellen eines Austrittsfensters des Lasersystems handeln kann, denen unterschiedliche Stellen der Hornhaut des Patienten, wenn dieser sich in definierter Position zum Lasersystem befindet, entsprechen. Erfindungsgemäß wird die Energie der Laserstrahlenpulse hinter den optischen Mitteln gemessen (bevorzugt vor der Zieleinrichtung, weil hinter der Zieleinrichtung die Laserstrahlenpulse an unterschiedlichen Stellen ausgekoppelt werden müssten), und es wird bei Erfüllung eines vorbestimmten Kriteriums die Abgabe der Laserpulse beendet, und zwar ungeachtet des Erfüllens (also des Erfüllseins oder Nichterfülltseins) der Ablationszielvorschrift, die Abgabe der Laserpulse wird also bei Erfüllung eines vorbestimmten Kriteriums auch bei noch nicht vollständig abgearbeiteter Tabelle beendet.

Wie oben bereits dargestellt, wird die Abgabe der Laserstrahlenpulse beendet, wenn die Gesamtenergie seit Beginn des Verfahrens einen vorbestimmten Schwellwert überschreitet. Sie kann zusätzlich beendet werden, wenn die Energie einzelner Laserstrahlenpulse einen Schwellwert überschreitet.

Die Schwellwerte können über eine Eingabe festgelegt werden. Sinnvoller ist es jedoch, das System selbst sich seinen Schwellwert festlegen zu lassen. Üblicherweise werden an dem Lasersystem bestimmte Einstellungen vorgenommen, welche patientenindividuell festgelegt werden. Insbesondere, wenn die Einstellungen erfolgen, während eine Rückmeldung an die einstellende Person erfolgt, z. B. durch Messung der Leistung von Laserpulsen in einem Testbetrieb, kann davon ausgegangen werden, dass die Laserstrahlenpulse zu Beginn des Betriebs des Lasersystems im Wesentlichen die gewünschte Energie haben. Dann kann das System den Schwellwert aufgrund der zu Beginn des Betriebs des Lasersystems (oder sogar bei einem Testlauf vor Beginn der eigentlichen Ablation, wenn die Laserstrahlenpulse z. B. in eine andere Richtung ausgekoppelt werden oder der Patient sich nicht im Lasersystem befindet) gewonnen Messergebnisse festgelegt werden. Dies gilt sowohl für den Schwellwert betreffend die Energie einzelner Laserstrahlenpulse als auch für den Schwellwert betreffend den gesamten Energieinhalt. Bei der Festlegung des letzteren Schwellwerts kann die Zahl der Schüsse gemäß der Ablationszielvorschrift, insbesondere der Schusstabelle, berücksichtigt werden.

Nachfolgend werden bevorzugte Ausführungsformen der Erfindung unter Bezug auf die Zeichnung veranschaulicht, wobei
- Figur 1: schematisch ein erfindungsgemäßes Lasersystem gemäß einer ersten Ausführungsform und
- Figur 2: schematisch ein erfindungsgemäßes Lasersystem gemäß einer zweiten Ausführungsform zeigt.

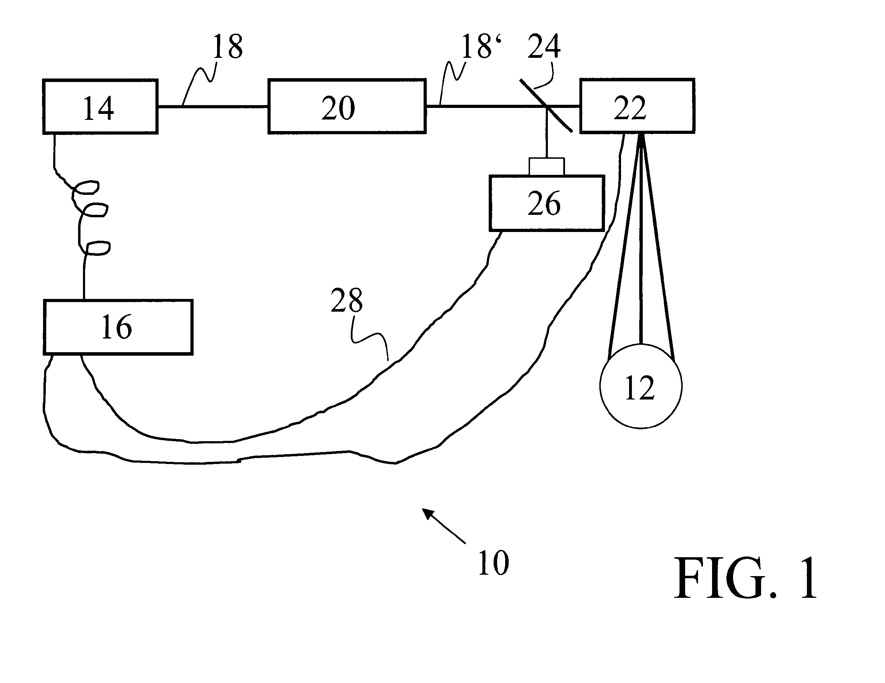

Ein in Figur 1 gezeigtes und dort im Ganzen mit 10 bezeichnetes Lasersystem dient zum Ablatieren von Hornhaut an einem Auge 12 eines Patienten. Die Ablation erfolgt mit Hilfe eines Laserstrahls, der von einer Laserquelle 14 erzeugt wird. Die Laserquelle 14 wird von einer zentralen Steuereinheit 16 angesteuert. In dieser zentralen Steuereinheit ist ein so genanntes Ablationsprofil zu dem Patientenauge 12 abgelegt, das beschreibt, welche Sollkontur das Patientenauge hat, d. h. wie es nach der Behandlung aussehen soll. Zu diesem Ablationsprofil werden Pulse von Laserstrahlen 18 erzeugt. Die Laserstrahlenpulse 18 werden von so genannten Strahlformungsoptiken 20 in an sich bekannter Weise geformt und einer Scannereinrichtung 22 zugeleitet. Unter Scannereinrichtung ist jede Einrichtung zu verstehen, die den von den Strahlformungsoptiken 20 abgegebenen Laserstrahl 18' in unterschiedliche Richtungen wahlweise auslenken kann. Typischerweise umfasst die Scannereinrichtung 22 einen ersten Spiegel für die Auslenkung in einer ersten Richtung (x-Richtung) und einen zweiten Spiegel für die Auslenkung in einer zweiten Richtung (y-Richtung). Diese Spiegel sind in der Figur nicht gezeigt. Die zentrale Steuereinheit 16 ist mit der Scannereinrichtung 22 verbunden und steuert diese an. Entsprechend dem Ablationsprofil wird durch die Steuereinheit 16 festgelegt, wie oft der Laserstrahl an welcher Stelle auf der Hornhaut des Patientenauges auftreffen soll. Unterschiedliche Auslenkungen der Scannereinrichtung 22 entsprechen hierbei dem Auftreffen des Lasers auf unterschiedlichen Stellen auf dem Patientenauge 12.

Vorliegend wird die Energie der Laserstrahlenpulse 18' gemessen, bevor sie auf die Scannereinrichtung 22 auftreffen. Hierzu ist im Strahlenweg der Laserstrahlenpulse 18' ein teildurchlässiger Spiegel 24 angeordnet, der einen definierten Anteil der Energie der Laserstrahlenpulse zu einem Energiesensor 26 sendet. Die von dem Energiesensor 26 gemessene Energie ist gemäß diesem definierten Anteil proportional zur Energie der Laserstrahlenpulse 18' bzw. dem nach dem Auskoppeln durch den teildurchlässigen Spiegel 24 verbleibenden und zur Scannereinrichtung 22 gelangenden Anteil dieser Energie. Der Energiesensor 26 ist über eine Verbindungsleitung 28 mit der zentralen Steuereinheit 16 verbunden, damit die Messwerte des Energiesensors 26 der zentralen Steuereinheit 16 zur Verfügung gestellt werden.

Die zentrale Steuereinheit 16 vergleicht die Messwerte mit zuvor festgelegten Schwellwerten. Ist der Energieinhalt eines einzelnen Laserstrahlenpulses 18' zu hoch, wird sofort der Betrieb der Laserquelle 14 unterbrochen. Andererseits summiert die Steuereinheit 16 auch die Messwerte auf. Sie vergleicht diese mit einem anhand des Ablationsprofils festgelegten Schwellwert. Wird dieser Schwellwert überschritten, veranlasst die zentrale Steuereinheit 16 ebenfalls ein Beenden des Betriebs der Laserquelle 14. Somit wird durch die zentrale Steuereinheit 16 verhindert, dass Laserstrahlenpulse mit zu hoher Energie auf das Auge 12 des Patienten gelangen. Auch die gesamte in das Auge eingetragene Energie bleibt so beschränkt.

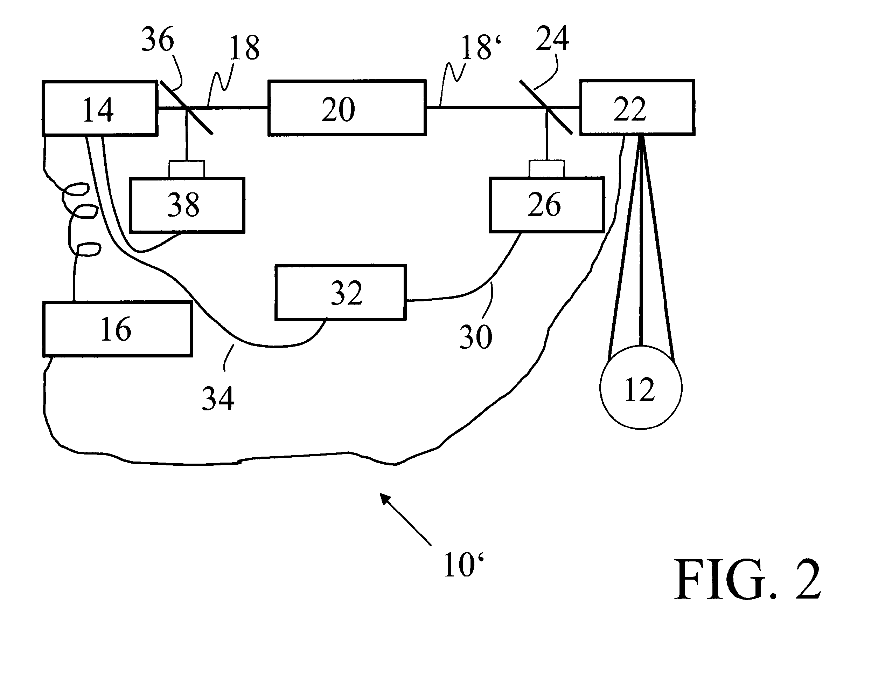

Lasersystem 10' gemäß Figur 2 unterscheidet sich von dem Lasersystem 10 nach Figur 1 dadurch, dass der Energiesensor 26 mit einer von der zentralen Steuereinheit 16 verschiedenen weiteren Steuereinheit 32 verbunden ist. Diese weitere Steuereinheit 32 kann auch als Energieauswerteeinheit bezeichnet werden. Die Energieauswerteeinheit 32 ist ihrerseits über eine Verbindungsleitung 34 mit der Laserquelle 14 verbunden. Die Energieauswerteeinheit 32 hat ausschließlich die Aufgabe, ein Unterbrechen des Betriebs der Laserquelle 14 zu bewirken. Sie prüft, ob die Energie der einzelnen Laserstrahlenpulse einen Schwellwert überschreitet. Genauso prüft sie auch, ob die gesamte Energie seit Beginn der Ablation einen Schwellwert überschreitet. Die Schwellwerte können hierbei in einem vorherigen Testlauf festgelegt worden sein, nachdem die Einstellungen an dem Lasersystem 10' vorgenommen worden sind. Während des Testlaufs kann eine Mehrzahl von Laserstrahlenpulsen abgegeben werden und deren Energieinhalt jeweils gemessen werden. Dann kann der Mittelwert der Energien berechnet werden. Der Schwellwert für das Beenden des Betriebs aufgrund des Überschreitens der Energie eines einzelnen Laserstrahlenpulses kann um einen vorgegebenen Prozentsatz über dem Mittelwert liegen oder nach einer sonstigen Rechenvorschrift aus diesem abgeleitet werden. Der Schwellwert für die Gesamtenergie kann aus einer typischen Zahl von Laserstrahlenpulsen bei einer Ablation und der mittleren Laserstrahlenpulsenergie abgeleitet werden. Es ist auch möglich, abweichend von der Darstellung in Figur 2 die zentrale Steuereinheit 16 mit der Energieauswerteeinheit 32 zu koppeln, damit letzterer die Gesamtzahl der geplanten Laserstrahlenpulse übermittelt werden kann. Im Übrigen ist es genauso möglich, auch den Energiesensor 26 zusätzlich zur Verbindungsleitung 30 über eine weitere Verbindungsleitung mit der zentralen Steuereinheit 16 zu koppeln. Ein weiterer Unterschied zwischen dem Lasersystem 10' nach Figur. 2 und dem Lasersystem 10 nach Figur 1 besteht darin, dass bei dem Lasersystem 10' auch die Energie der Laserstrahlenpulse 18 nach ihrem Auskoppeln aus der Laserquelle 14 gemessen wird, bevor sie die Strahlformungsoptiken 20 durchlaufen. Hierzu wird ein teildurchlässiger Spiegel 36 bereitgestellt, der einen Teil der Energie zu einem Energiesensor 38 auskoppelt, welcher mit der Laserquelle verbunden ist. In an sich bekannter Weise wird der von dem Sensor 38 aufgenommene Messwert zur Regelung der Leistung des Lasers der Laserquelle 14 verwendet. Die Einrichtung zum Messen mit dem teildurchlässigen Spiegel 36 und dem Energiesensor 38 kann auch bei der Ausführungsform aus Figur 1 vorgesehen sein. Umgekehrt kann sie bei der Ausführungsform aus Figur 2 auch entfallen.

## Patentansprüche

1. Lasersystem (10, 10') zum Ablatieren von Hornhaut an einem Patientenauge (12), mit einer Laserquelle (14), die im Betrieb Laserstrahlenpulse (18) aussendet, die vor ihrem Austreten aus dem Lasersystem (10, 10') durch optische Mittel (20) verändert werden, mit einer Einrichtung (24, 26) zum Messen der Energie eines aus den optischen Mitteln (20) austretenden Laserstrahlenpulses (18'), die mit einer Steuereinheit (16,32), welche zum Beenden des Betriebs der Laserquelle (12) ausgelegt ist, gekoppelt ist, und wobei die Steuereinrichtung entweder dazu ausgelegt ist, sowohl die Laserquelle (14), als auch eine Zieleinrichtung (22), die in Abhängigkeit von den Steuerbefehlen Laserstrahlenpulse nacheinander auf unterschiedliche Stellen lenkt, gemäß einer zuvor definierten Tabelle anzusteuern und aufgrund vorbestimmter Ergebnisse einer Messung durch die Einrichtung (24, 26) zum Messen von einer Steuerung gemäß der Tabelle abzuweichen, indem der Betrieb der Laserquelle (14) beendet wird, oder wobei die Steuereinrichtung (32) dazu ausgelegt ist, ausschließlich solche Steuerbefehle an die Laserquelle (14) zu senden, mit denen ein Beenden des Betriebs der Laserquelle (14) erfolgt, **dadurch gekennzeichnet, dass** die Steuereinrichtung (16, 32) dazu ausgelegt ist, ein Beenden des Betriebs der Laserquelle (14) zu bewirken, wenn die Summe der Energie sämtlicher seit einem Beginn einer Laserablation abgegebenen Laserstrahlenpulse (18') einen vorbestimmten Schwellwert überschreitet.

2. Lasersystem (10, 10') nach Anspruch 1,
**dadurch gekennzeichnet,**
**dass** die Steuereinrichtung (16, 32) dazu ausgelegt ist, ein Beenden des Betriebs der Laserquelle auch zu bewirken, wenn die Energie zumindest eines vorbestimmten Teils der zuletzt abgegebenen Laserstrahlenpulse (18') einen Schwellwert überschreitet.

## Claims

1. A laser system (10, 10') for ablating cornea on a patient's eye (12), including a laser source (14) emitting laser beam pulses (18) in operation, which are modified by optical means (20) before their exit from the laser system (10, 10'), including a device (24, 26) for measuring the energy of a laser beam pulse (18') exiting the optical means (20), which is coupled to a controller (16, 32) adapted to terminate the operation of the laser source (12), and wherein either the controller is adapted to drive both the laser source (14) and a targeting device (22) successively directing laser beam pulses to different locations depending on the control commands, according to a previously defined table and to deviate from control according to the table due to predetermined results of a measurement by the device (24, 26) for measuring by terminating the operation of the laser source (14), or wherein the controller (32) is adapted to send exclusively such control commands to the laser source (14), by which termination of the operation of the laser source (14) is effected, **characterized in that** the controller (16, 32) is adapted to effect termination of the operation of the laser source (14) if the sum of all of the energy of all of the laser beam pulses (18') emitted since beginning of laser ablation exceeds a predetermined threshold value.

2. The laser system (10, 10') according to claim 1,
**characterized in that**
the controller (16, 32) is adapted to also effect termination of the operation of the laser source if the energy of at least a predetermined portion of the lastly emitted laser beam pulses (18') exceeds a threshold value.

## Revendications

1. Système laser (10, 10'), destiné à l'ablation de la cornée de l'oeil d'un patient (12), avec une source laser (14), qui émet, en fonctionnement, des impulsions de rayon laser (18), qui sont modifiées, avant leur émergence du système laser (10, 10'), par le biais de moyens optiques (20), avec un dispositif (24, 26), destiné à mesurer l'énergie d'une impulsion de rayon laser (18') qui émerge des moyens optiques (20), qui est couplée avec une unité de commande (16, 32), laquelle est conçue pour mettre fin à l'exploitation de la source laser (12) et moyennant quoi le dispositif de commande soit est conçu pour commander tant la source laser (14) qu'un dispositif de visée (22), qui dirige, en fonction des instructions de commande, les impulsions de rayon laser successivement sur différents points, conformément à un tableau défini au préalable et pour s'écarter d'une commande conformément au tableau, sur la base de résultats prédéfinis d'une mesure, par le biais du dispositif (24, 26) de mesure, en mettant fin à l'exploitation de la source laser (14), soit moyennant quoi le dispositif de commande (32) est conçu, pour émettre exclusivement de telles instructions de commande à destination de la source laser (14), qui entraînent une cessation de l'exploitation de la source laser (14),
**caractérisé en ce que**
le dispositif de commande (16, 32) est conçu pour provoquer une cessation de l'exploitation de la source laser (14), lorsque la somme de l'énergie de toutes les impulsions de rayon laser (18'), délivrées depuis le début d'une ablation au laser, dépasse une valeur seuil prédéfinie.

2. Système laser (10, 10') selon la revendication 1,
**caractérisé en ce que**
le dispositif de commande (16, 32) est conçu pour provoquer aussi une cessation de l'exploitation de la source laser, lorsque l'énergie dépasse une valeur seuil d'au moins une partie prédéfinie des impulsions de rayon laser (18'), délivrées en dernier.
